# EUROPEAN PATENT APPLICATION

(11) **EP 1 916 247 A1**
(43) Date of publication of application: **30.04.2008**
(21) Application number: 06780910.3
(22) Date of filing: 04.07.2006
(51) Int. Cl.: C07D 413/06, A61K 31/42, A61P 1/00, A61P 3/04, A61P 3/06, A61P 3/10, A61P 9/00, A61P 9/10, A61P 11/00, A61P 15/00, A61P 17/00, A61P 25/00, A61P 25/28, A61P 29/00, A61P 35/00, A61P 43/00

(54) **ACTIVATOR OF PEROXISOME PROLIFERATOR ACTIVATED RECEPTOR**

(30) Priority: 06.07.2005 JP 2005197484
(71) Applicant: Nippon Chemiphar Co., Ltd., Chiyoda-ku Tokyo 1010032 (JP)
(72) Inventor: SAKUMA, Shogo, 3420041 (JP); MOCHIDUKI, Nobutaka, 2701166 (JP); TAKAHASHI, Rie, 3410005 (JP); HIRAI, Toshitake, 2780036 (JP); YAMAKAWA, Tomio, 2770884 (JP); MASUI, Seiichiro, 3620072 (JP)
(74) Representative: Weber-Quitzau, Martin
(86) International application number: PCT/JP2006/313636
(87) International publication number: WO 2007/004733

(57) **Abstract**

A compound having the following formula (II) or its salt is used as an activator for PPAR δ: 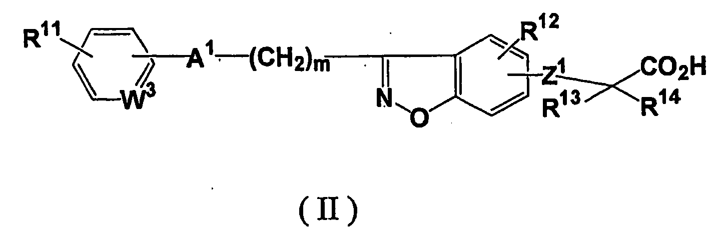 [in which W³ is a nitrogen atom or CH; Z¹ is an oxygen atom or a sulfur atom; each of R¹¹ and R¹² is a hydrogen atom, a halogen atom, a C₁-C₈ alkyl group, a C₁-C₈ alkoxy group, a C₁-C₈ alkyl group having a halogen substituent, or the like; each of R¹³ and R¹⁴ is a hydrogen atom or a C₁-C₈ alkyl group; A¹ is pyrazole or thiophene which may have a substituent; and m is an integer of 2 to 4].

## Description

### [FIELD OF THE INVENTION]

The present invention relates to an activator for peroxisome proliferator activated receptor (PPAR) δ.

### [BACKGROUND OF THE INVENTION]

As for the peroxisome proliferator activated receptor (PPAR), it is known that there are three subtypes such as PPARα, PPARγ and PPARδ. --- Proc. Natl. Acad. Sci. USA, 91, p7335-7359, 1994 (Non-Patent Publication 1).

Until now, a transcription-activating function, a glycemic index-depressing function, and a lipid metabolism-improving function have been reported on each subtype of PPAR.

For instance, WO 97/28115 (Patent Publication 1) describes use of L-165041 (Merck) as a diabetes treatment medicine and an anti-obesity medicine; WO 99/04815 (Patent Publication 2) describes that YM-16638 (Yamanouchi) has a serum cholesterol-depressing function and an LDL cholesterol-depressing function; and WO 2004/007439 (Patent Publication 3) describes use of biaryl derivatives as medicines for enhancing a blood HDL. In addition, a large number of patent applications, namely, WO 01/40207 (Patent Publication 4: GW-590735, GSK), WO 2004/63166 (Patent Publication 5: pyrazole derivatives, Lilly), WO 02/092590 (Patent Publication 6: thiophene derivatives, GSK), WO 03/099793 (Patent Publication 7: azole derivatives, Takeda), and WO 2004/063190 (Patent Publication 8, benzothiophene derivatives for treating syndrome X, Eli Lilly) have been filed.

WO 01/603 (Patent Publication 9) describes that GW-501516 (GSK) having the following formula: 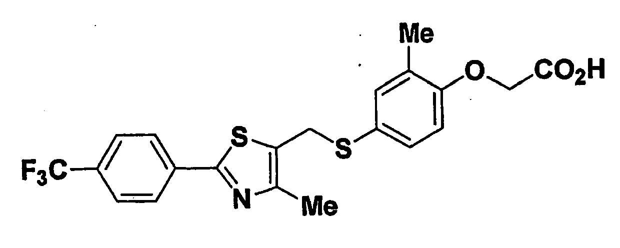 is being studied as a lipid metabolism-improving medicine.

The present inventors have already filed patent applications (WO 01/79197 (Patent Publication 10), WO 03/033493 (Patent Publication 11), etc.) for compounds having a benzisoxazole ring which have a PPAR δ transcription-activating function.

There are clear structural differences between the above-illustrated GW-501516 and the compounds of the present invention which are represented by the below-illustrated formula (I) or (II). Further, the latter compounds are not clearly described in the aforementioned patent publications.

### [DISCLOSURE OF THE INVENTION]

The invention has an object to provide compounds having the following formula (I) or (II), which have an activating function for peroxisome proliferator-activated receptor δ.

In one aspect, the invention resides in compounds having the following formula (I) or salts thereof: 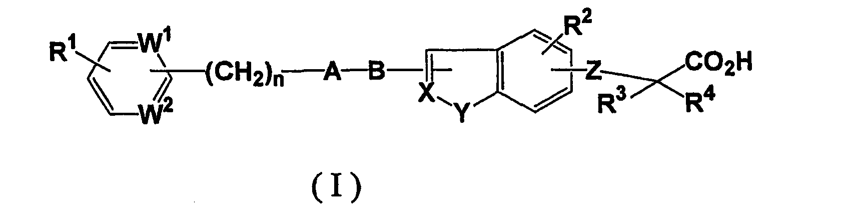 [in which
each of W¹ and W² independently represents a nitrogen atom or CH,
X represents a nitrogen atom or CH,
Y represents an oxygen atom or a sulfur atom,
Z represents a bond, an oxygen atom, a sulfur atom or NR⁵, in which R⁵ represents a hydrogen atom or an alkyl group having 1 to 8 carbon atoms,
each of R¹ and R² independently represents a hydrogen atom, a halogen atom, a hydroxyl group, a nitro group, an amino group, an alkyl group having 1 to 8 carbon atoms, a 3- to 7-membered cycloalkyl group, an alkenyl group having 2 to 8 carbon atoms, an alkynyl group having 2 to 8 carbon atoms, an alkoxy group having 1 to 8 carbon atoms, an alkyl group having 1 to 8 carbon atoms and a 3- to 7-membered cycloalkyl substituent, an alkyl group having 1 to 8 carbon atoms and a halogen substituent, an alkoxy group having 1 to 8 carbon atoms and a halogen substituent, an aryl group having 6 to 10 carbon atoms, 5- or 6-membered heterocyclic group, an aralkyl group having an aryl moiety of 6 to 10 carbon atoms and an alkylene moiety of 1 to 8 carbon atoms, or an alkyl group having 1 to 8 carbon atoms and a 5- or 6-membered heterocyclic substituent,
each of R³ and R⁴ independently represents a hydrogen atom, an alkyl group having 1 to 8 carbon atoms, or an alkyl group having 1 to 8 carbon atoms and a halogen substituent,
A represents a 5-membered hetero ring selected from the group consisting of pyrazole, thiophene, furan, isoxazole, isothiazole and pyrrole, in which the 5-membered hetero ring may have a substituent selected from the group consisting of a halogen atom, a hydroxyl group, a nitro group, an amino group, an alkyl group having 1 to 8 carbon atoms, a 3- to 7-membered cycloalkyl group, an alkenyl group having 2 to 8 carbon atoms, an alkynyl group having 2 to 8 carbon atoms, an alkoxy group having 1 to 8 carbon atoms, an alkyl group having 1 to 8 carbon atoms and a 3- to 7-membered cycloalkyl substituent, an alkyl group having 1 to 8 carbon atoms and a halogen substituent, an alkoxy group having 1 to 8 carbon atoms and a halogen substituent, an aryl group having 6 to 10 carbon atoms, a 5- or 6-membered heterocyclic group, an aralkyl group having an aryl moiety of 6 to 10 carbon atoms and an alkylene moiety of 1 to 8 carbon atoms, and an alkyl group having 1 to 8 carbon atoms and a 5- or 6-membered heterocyclic substituent,
B represents a bond or an alkylene chain having 1 to 8 carbon atoms which may have a substituent selected from the group consisting of an alkyl group having 1 to 8 carbon atoms, a 3- to 7-membered cycloalkyl group, an alkoxy group having 1 to 8 carbon atoms and a halogen substituent and further may have a double or triple bond,
and
n is an integer of 0 to 3].

In another aspect, the invention resides in a compound having the following formula (II) or a salt thereof: 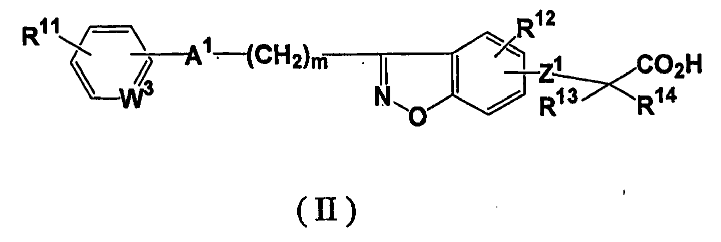 [in which
W³ represents a nitrogen atom or CH,
Z¹ represents an oxygen atom or a sulfur atom,
each of R¹¹ and R¹² independently represents a hydrogen atom, a halogen atom, a hydroxyl group, a nitro group, an amino group, an alkyl group having 1 to 8 carbon atoms, an alkoxy group having 1 to 8 carbon atoms, an alkyl group having 1 to 8 carbon atoms and a halogen substituent, or an alkoxy group having 1 to 8 carbon atoms and a halogen substituent,
each of R¹³ and R¹⁴ independently represents a hydrogen atom or an alkyl group having 1 to 8 carbon atoms,
A¹ represents pyrazole or thiophene which may have a substituent selected from the group consisting of a halogen atom, a hydroxyl group, a nitro group, an amino group, an alkyl group having 1 to 8 carbon atoms, an alkoxy group having 1 to 8 carbon atoms, an alkyl group having 1 to 8 carbon atoms and a halogen substituent, or an alkoxy group having 1 to 8 carbon atoms and a halogen substituent, and
m is an integer of 2 to 4].

In a further aspect, the invention resides in an activator for peroxisome proliferator activated receptor δ containing a compound of the formulas (I) or (II) as an effective component.

### [PREFERRED EMBODIMENTS OF THE INVENTION]

The invention is described below in detail.

In the formula (I), the alkyl group having 1 to 8 carbon atoms for R¹, ,R², R³, R⁴, R⁵, a substituent possibly attached to the 5-membered hetero ring of A, and a substituent possibly attached to the alkylene chain having 1 to 8 carbon atoms can be methyl, ethyl, propyl, isopropyl, butyl, i-butyl, t-butyl, pentyl, or hexyl.

The alkenyl group having 2 to 8 carbon atoms for R¹, R² and a substituent possibly attached to the 5-membered hetero ring of A can be vinyl or allyl.

The alkynyl group having 2 to 8 carbon atoms for R¹, R² and a substituent possibly attached to the 5-membered hetero ring of A can be propargyl.

The 3- to 7-membered cycloalkyl group for R¹, R², a substituent possibly attached to the 5-membered hetero ring of A, and a substituent possibly attached to the alkylene chain having 1 to 8 carbon atoms can be cyclopentyl or cyclohexyl.

The alkoxy group having 1 to 8 carbon atoms for R¹, R², a substituent possibly attached to the 5-membered hetero ring of A, and a substituent possibly attached to the alkylene chain having 1 to 8 carbon atoms can be methoxy, ethoxy, propoxy, isopropoxy, butoxy, i-butoxy, t-butoxy, pentyloxy, or hexyloxy.

The halogen atom for R¹, R², a substituent possibly attached to the 5-membered hetero ring of A, and a substituent possibly attached to the alkylene chain having 1 to 8 carbon atoms can be fluorine, chlorine, or bromine.

The alkyl group having 1 to 8 carbon atoms and a halogen substituent for R¹, R², R⁵, and a substituent possibly attached to the 5-membered hetero ring of A can be methyl, ethyl, propyl, isopropyl, butyl or t-butyl which has 1 to 3 halogen substituents such as fluorine, chlorine or bromine. Preferred are trifluoromethyl, chloromethyl, 2-chloroethyl, 2-bromoethyl, and 2-fluoroethyl.

The alkoxy group having 1 to 8 carbon atoms and a halogen substituent for R¹, R², and a substituent possibly attached to the 5-membered hetero ring of A can be methoxy, ethoxy, propoxy, isopropoxy, butyloxy or t-butyloxy which has 1 to 3 halogen substituents such as fluorine, chlorine or bromine. Preferred are trifluoromethyloxy, chloromethyloxy, 2-chloroethyloxy, 2-bromoethyloxy, and 2-fluoroethyloxy.

The aryl group having 6 to 10 carbon atoms for R¹, R², and a substituent possibly attached to the 5-membered hetero ring of A can be phenyl.

The 5- or 6-membered heterocyclic group for R¹, R², and a substituent possibly attached to the 5-membered hetero ring of A can be pyridyl.

The alkyl group having 1 to 8 carbon atoms and 3- to 7-membered cycloalkyl group for R¹, R², and a substituent possibly attached to the 5-membered hetero ring of A can be methyl, ethyl, propyl, isopropyl, butyl, i-butyl, t-butyl, pentyl, or hexyl which has a cyclopropyl substituent, a cyclopentyl substituent, or a cyclohexyl substituent.

The aralkyl having an aryl moiety of 6 to 10 carbon atoms and an alkylene moiety of 1 to 8 carbon atoms for R¹, R², and a substituent possibly attached to the 5-membered hetero ring of A can be benzyl or phenethyl.

The alkyl group having 1 to 8 carbon atoms and 5- or 6-membered heterocyclic group for R¹, R², and a substituent possibly attached to the 5-membered hetero ring of A can be methyl, ethyl, propyl, isopropyl, butyl, i-butyl, t-butyl, pentyl, or hexylwhich has a pyridyl substituent.

The 5-membered hetero ring which may have a substituent for A preferably is pyrazole or thiophene which possibly has a substituent. More preferred is pyrazole possibly having a substituent.

The alkylene chain having 1 to 8 carbon atoms which may have a substituent for B preferably is an alkylene chain having 1 to 4 carbon atoms. More preferred are an ethylene chain and a propylene chain.

n preferably is 0.

In the formula (II), the halogen atom, alkyl group having 1 to 8 carbon atoms, alkoxy group having 1 to 8 carbon atoms, alkyl group having 1 to 8 carbon atoms and a halogen substituent, and alkoxy group having 1 to 8 carbon atoms and a halogen substituent for R¹¹ and R¹² can be those described hereinbefore for R¹ and R² of the formula (I).

The alkyl group having 1 to 8 carbon atoms for R¹³ and R¹⁴ can be those described hereinbefore for R³ and R⁴ of the formula (I).

The halogen atom, alkyl group having 1 to 8 carbon atoms, alkoxy group having 1 to 8 carbon atoms, alkyl group having 1 to 8 carbon atoms and a halogen substituent, and alkoxy group having 1 to 8 carbon atoms and a halogen substituent which is possibly attached to
pyrazole or thiophene for A¹ in the formula (II) can be those described hereinbefore for the substituents possibly attached to the 5-membered hetero ring of A of the formula (I).

As for R¹ of the formula (I) and R¹¹ of the formula (II), the benzene ring or the like can have 1 to 3 number of R¹ or R¹¹ which are the same or different from each other. In other words, the benzene ring or the like can have 1 to 3 substituents other than a hydrogen atom.

As for R² of the formula (I) and R¹² of the formula (II), the benzene ring of the benzisoxazole ring or the like can have 1 to 3 number of R² or R¹² which are the same or different from each other. In other words, the benzene ring of the benzisoxazole ring or the like can have 1 to 3 substituents other than a hydrogen atom.

The substituent possibly attached to the 5-membered hetero ring for A of the formula (I) and the substituent possibly attached to pyrazole or thiophene for A¹ of the formula (II) can be present in 1 or 2 number which can be the same or different from each other.

Preferred examples of the compounds according to the invention are set forth below.
(1) The compound or a salt thereof represented by the formula (I), in which each of W¹ and W² represents CH.
(2) The compound or a salt thereof represented by the formula (I), in which W¹ represents CH and W² represents a nitrogen atom.
(3) The compound or a salt thereof represented by the formula (I) or according to (1) or (2) above, in which X represents a nitrogen atom.
(4) The compound or a salt thereof represented by the formula (I) or according to (1) or (2) above, in which X represents a nitrogen atom and Y represents an oxygen atom.
(5) The compound or a salt thereof represented by the formula (I) or according to (1) or (2) above, in which X represents CH and Y represents an oxygen atom.
(6) The compound or a salt thereof represented by the formula (I) or according to any one of (1) to (5) above, in which Z represents an oxygen atom or a sulfur atom.
(7) The compound or a salt thereof represented by the formula (I) or according to any one of (1) to (6) above, in which each of R¹ and R² independently represents a hydrogen atom, a halogen atom, a hydroxyl group, a nitro group, an amino group, an alkyl group having 1 to 8 carbon atoms, an alkoxy group having 1 to 8 carbon atoms, an alkyl group having 1 to 8 carbon atoms and a halogen substituent, or an alkoxy group having 1 to 8 carbon atoms and a halogen substituent.
(8) The compound or a salt thereof represented by the formula (I) or according to any one of (1) to (7) above, in which each of R³ and R⁴ independently represents a hydrogen atom or an alkyl group having 1 to 8 carbon atoms.
(9) The compound or a salt thereof represented by the formula (I) or according to any one of (1) to (8) above, in which A represents pyrazole, thiophene or furan which may have a substituent selected from the group consisting of a halogen atom, a hydroxyl group, a nitro group, an amino group, an alkyl group having 1 to 8 carbon atoms, a 3- to 7-membered cycloalkyl group, an alkenyl group having 2 to 8 carbon atoms, an alkynyl group having 2 to 8 carbon atoms, an alkoxy group having 1 to 8 carbon atoms, an alkyl group having 1 to 8 carbon atoms and a 3- to 7-membered cycloalkyl substituent, an alkyl group having 1 to 8 carbon atoms and a halogen substituent, an alkoxy group having 1 to 8 carbon atoms and a halogen substituent, an aryl group having 6 to 10 carbon atoms, a 5- or 6-membered heterocyclic group, an aralkyl group having an aryl moiety of 6 to 10 carbon atoms and an alkylene moiety of 1 to 8 carbon atoms, and an alkyl group having 1 to 8 carbon atoms and a 5- or 6-membered heterocyclic substituent.
(10) The compound or a salt thereof represented by the formula (I) or according to any one of (1) to (8) above, in which A represents pyrazole, thiophene or furan which may have a substituent selected from the group consisting of a halogen atom, a hydroxyl group, a nitro group, an amino group, an alkyl group having 1 to 8 carbon atoms, an alkoxy group having 1 to 8 carbon atoms, an alkyl group having 1 to 8 carbon atoms and a halogen substituent, or an alkoxy group having 1 to 8 carbon atoms and a halogen substituent.
(11) The compound or a salt thereof represented by the formula (I) or according to any one of (1) to (8) above, in which A represents pyrazole which may have a substituent selected from the group consisting of a halogen atom, a hydroxyl group, a nitro group, an amino group, an alkyl group having 1 to 8 carbon atoms, an alkoxy group having 1 to 8 carbon atoms, an alkyl group having 1 to 8 carbon atoms and a halogen substituent, and an alkoxy group having 1 to 8 carbon atoms and a halogen substituent.
(12) The compound or a salt thereof represented by the formula (I) or according to any one of (1) to (11) above, in which B represents an alkylene chain having 2 to 4 carbon atoms.
(13) The compound or a salt thereof represented by the formula (I) or according to any one of (1) to (12) above, in which n is 0.
(14) The compound or a salt thereof represented by the formula (II), in which W³ represents CH.
(15) The compound or a salt thereof represented by the formula (II) or according to (14) above, in which A¹ represents pyrazole which may have a substituent selected from the group consisting of a halogen atom, a hydroxyl group, a nitro group, an amino group, an alkyl group having 1 to 8 carbon atoms, an alkoxy group having 1 to 8 carbon atoms, an alkyl group having 1 to 8 carbon atoms and a halogen substituent, or an alkoxy group having 1 to 8 carbon atoms and a halogen substituent.
(16) The compound or a salt thereof represented by the formula (II) or according to (14) or (15) above, in which m is 2 or 3.

The compound of the formula (I) or (II) can be in the form of a pharmacologically acceptable salt such as a salt of an alkali metal such as sodium, potassium, or lithium.

The compounds of the invention can be present in the optically active forms, and in the form of optical isomers such as compounds of a racemic form or geometric isomers such as compounds of a cis- or trans form.

The schemes for synthesis of the compounds of the invention having the formula (I) are illustrated below.

### [Synthesis Process 1]

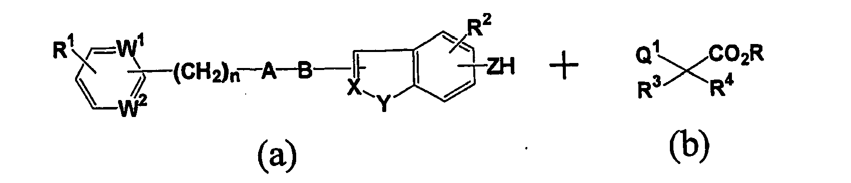

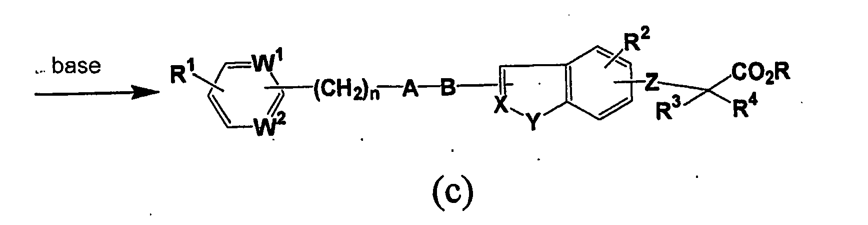

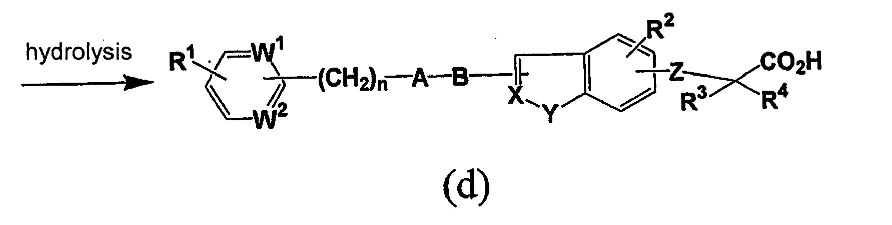

[in the formulas, Q¹ represents a halogen atom such as bromine, R represents a lower alkyl group, and each of W¹, W², A, B, R¹, R², R³, R⁴, X, Y, Z and n has the same meaning as those described hereinbefore.]

The ester compound of the formula (c) can be obtained by reacting the compound of the formula (a) and the compound of the formula (b) in an inert solvent such as 2-butanone in the presence of a base such as potassium carbonate. The ester compound of the formula (c) obtained as above can be subjected to hydrolysis in the presence of a base such as sodium hydroxide, potassium carbonate or lithium hydroxide, to give the compound of the invention having the formula (d).

The starting compound, i.e., the compound of the formula (a), can be obtained by the following process:

### [Synthesis process for the starting compound (B=ethylene, Z=oxygen)]

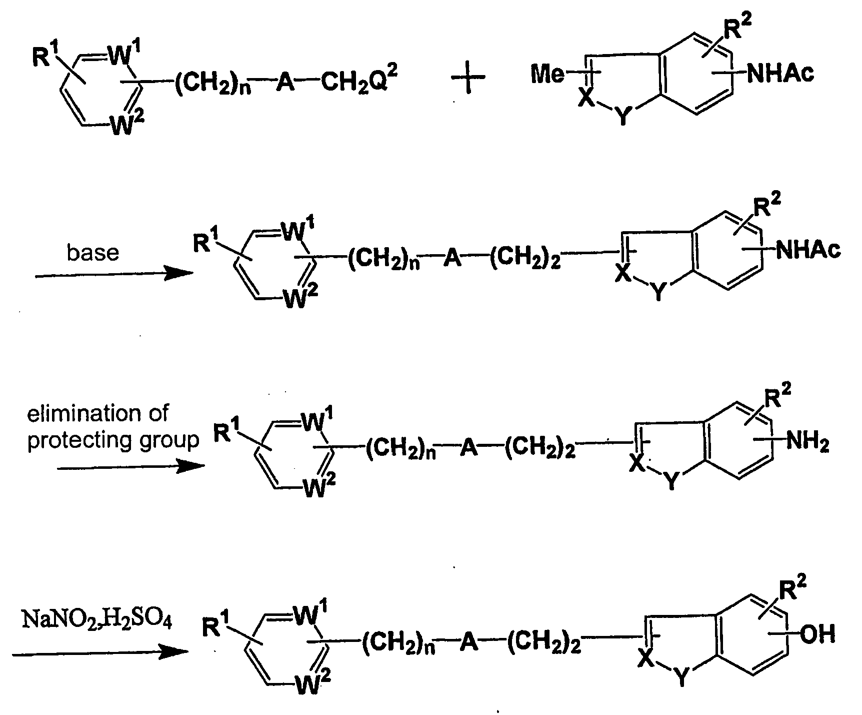

[in the formulas, Q² represents a halogen atom such as chlorine, Ac represents acetyl, and each of W¹, W², A, R¹, R², X, Y and n has the same meaning as those described hereinbefore].

The compounds of the invention can be prepared by the following Synthesis Processes 2 and 3:

### [Synthesis Process 2]

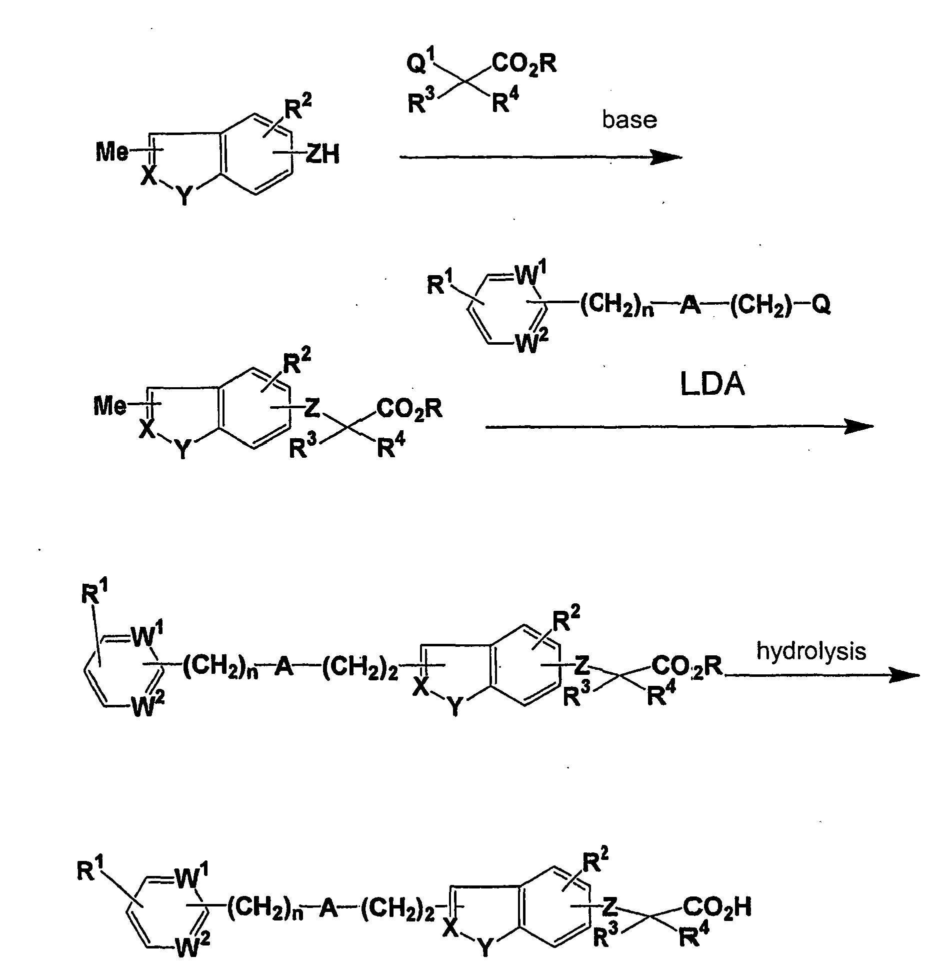

[in the formulas, Q¹ represents a halogen atom such as bromine, R represents a lower alkyl group, and each of W¹, W², A, R¹, R², R³, R⁴, X, Y, Z and n has the same meaning as those described hereinbefore].

### [Synthesis Process 3]

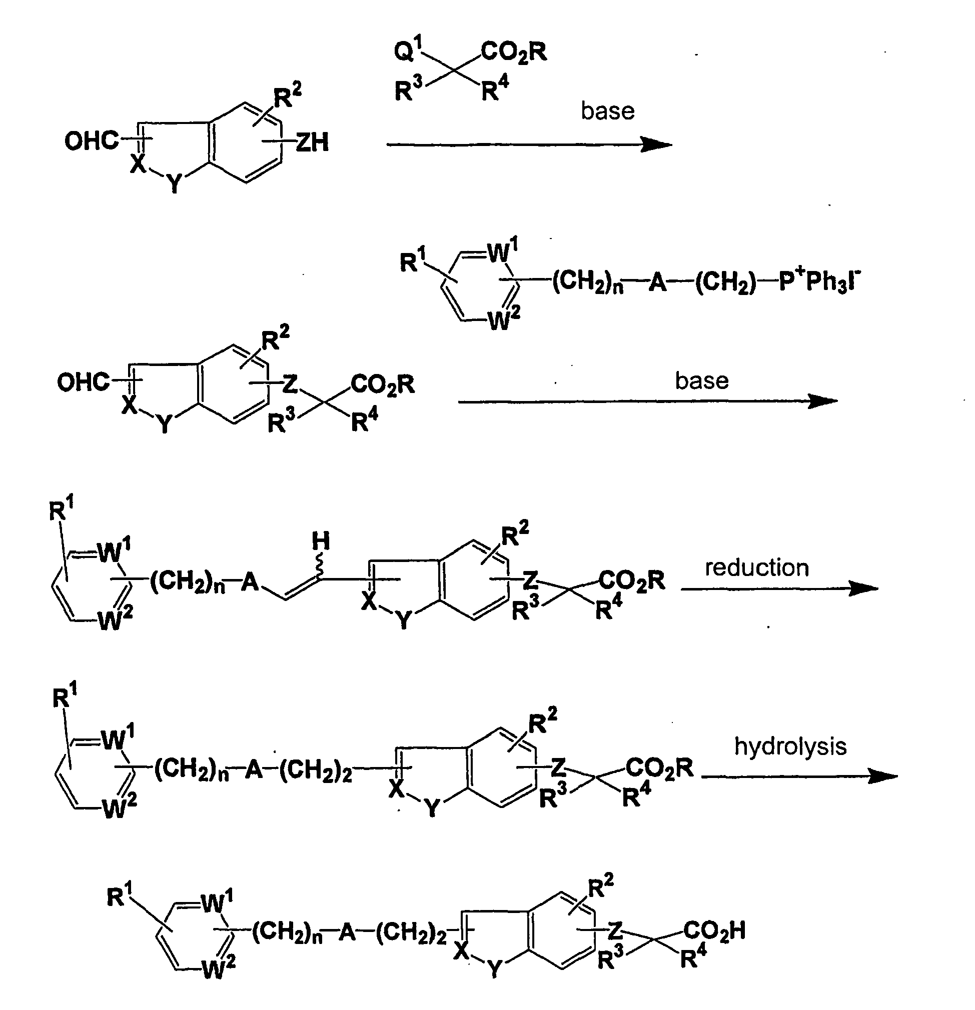

[in the formulas, R represents a lower alkyl group, and each of W¹, W², A, R¹, R², R³, R⁴, X, Y, Z and n has the same meaning as those described hereinbefore].

In addition, the compound of the invention having the formula (I) or (II) can be prepared by referring to the after-described working examples and the aforementioned patent publication and other publications.

Examples of the compounds according to the invention are described in the below-given tables.

### (1) Compounds represented by the following formula:

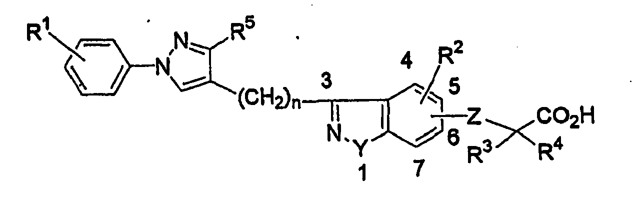

[in which Y, Z, R¹, R², R³, R⁴, R⁵, and n are described in Tables 1 and 2].

**Table 1**

| Y | Z | R¹ | R² | R³/ R⁴ R⁵ | n | |
|---|---|---|---|---|---|---|
| O | O(6) | 4-CF₃ | 5-Me | Me/Me | iPr | 2 |
| O | O(6) | 4-CF₃ | 5-Me | H/H | iPr | 2 |
| O | O(6) | 4-Me | 5-Me | Me/Me | iPr | 2 |
| O | O(6) | 4-CF₃ | 5-Me | Me/Me | hexyl | 2 |
| O | O(6) | 4-CF₃ | 5-Me | H/H | hexyl | 2 |
| O | O(6) | 4-CF₃ | 5-Me | Me/Me | iPr | 3 |
| O | O(6) | 4-CF₃ | 5-Me | Me/Me | iPr | 4 |
| S | O(6) | 4-CF₃ | 5-Et | Me/Me | hexyl | 2 |
| S | S(6) | 3-Me | 5-nPr | H/H | Me | 3 |
| O | O(5) | 3,4-Cl | 7-OMe | H/H | allyl | 3 |

**Table 2**

| Y | Z | R¹ | R² | R³/R⁴ R⁵ | n | |
|---|---|---|---|---|---|---|
| O | O(6) | 4-OCF₃ | 5-Et | H/H | allyl | 2 |
| S | O(5) | 3-Cl, 4-CF₃ | 6-Me Me/Me | iPr | 2 | |
| S | O(5) | 3-Cl, 4-CF₃ | 6-Me H/H | iPr | 2 | |
| O | O(5) | 3-F,4-Cl | 6-allyl | Et/H | hexyl | 3 |
| O | O(6) | 4-CF₃ | 5-Me | Me/Me | octyl | 2 |
| O | O(6) | 4-CF₃ | 5-Me | Me/Me | pentyl | 2 |
| O | O(6) | 4-CF₃ | 5-Me | Me/Me | OMe | 2 |
| O | O(6) | 4-CF₃ | 5-Me | Me/Me | CF₃ | 2 |
| O | O(6) | 4-CF₃ | 5-Me | Me/Me | phenyl | 2 |
| O | O(6) | 4-CF₃ | 5-Me | Me/Me | phenylethyl | 2 |

### (2) Compounds represented by the following formula:

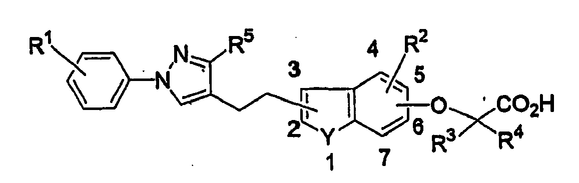

[in which Y, R¹, R², R³, R⁴ and R⁵ are described in Table 3; in Table 3, P-1 means a substitution position of O, and P-2 means a substitution position of (CH₂)₂].

**Table 3**

| Y | R¹ | R² | R³/R⁴ R⁵ | P-1 | P-2 | |
|---|---|---|---|---|---|---|
| O | 4-CF₃ | 5-Me | Me/Me | iPr | 6 | 3 |
| O | 4-CF₃ | 5-Me | H/H | iPr | 6 | 2 |
| S | 4-OCF₃ | 7-OMe | Me/Me | hexyl | 5 | 2 |
| S | 4-OCF₃ | 5-Et | Me/Me | allyl | 6 | 3 |
| S | 4-OCF₃ | 5-Et | Me/Me | allyl | 6 | 2 |
| O | 4-CF₃ | 6-Me | Me/Me | CH₂OBu | 5 | 3 |
| O | 3-F, 4-Cl | 6-allyl | Et/H | hexyl | 5 | 3 |
| S | 3-F,4-Cl | 6-allyl | Et/H | hexyl | 5 | 3 |

### (3) Compounds represented by the following formula:

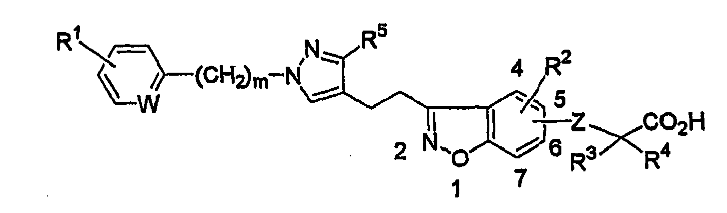

[in which W, Z, R¹, R², R³, R⁴, R⁵ and m are described in Table 4].

**Table 4**

| W | Z | R¹ | R² | R³/R⁴ R⁵ | m | |
|---|---|---|---|---|---|---|
| CH | O(6) | 3-CF₃ | 5-Me | Me/Me | iPr | 1 |
| CH | O(6) | 3-CF₃ | 5-Me | H/H | iPr | 1 |
| CH | O(6) | 3-CF₃ | 5-Me | Me/Me | iPr | 2 |
| CH | O(6) | 3-Me | 5-Me | Me/Me | iPr | 1 |
| N | O(6) | 3-CF₃ | 5-Me | Me/Me | iPr | 0 |
| N | O(5) | 3-Me | 6-Et | H/H | hexyl | 0 |
| N | O(5) | 3-Me | 7-Me | Me/Me | Me | 0 |
| N | S(6) | 2,4-Cl | 5-H | Me/Me | nBu | 0 |

### (4) Compounds represented by the following formula:

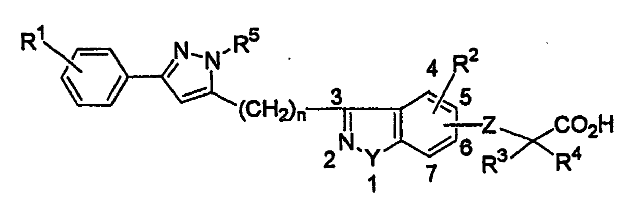

[in which Y, Z, R¹, R², R³, R⁴, R⁵ and n are described in Table 5] .

**Table 5**

| Y | Z | R¹ | R² | R³/R⁴ R⁵ | n | |
|---|---|---|---|---|---|---|
| O | O(6) | 4-CF₃ | 5-Me | Me/Me | iPr | 2 |
| O | O(6) | 4-CF₃ | 5-Me | H/H | iPr | 2 |
| O | O(6) | 4-CF₃ | 5-Me | Me/Me | hexyl | 2 |
| O | O(6) | 4-CF₃ | 5-Me | H/H | hexyl | 2 |
| O | O(6) | 4-Me | 5-Et | H/H | iPr | 2 |
| O | O(5) | 4-Me | 6-Me | Me/Me | nBu | 3 |
| O | O(5) | 3,4-Me | 6-OMe | H/H | hexyl | 2 |
| O | O(5) | 4-Me | 6-Me | Me/Me | allyl | 2 |
| O | S(5) | 4-Me | 6-Me | Me/Me | allyl | 2 |
| S | O(6) | 4-CF₃ | 5-Me | Me/Me | iPr | 2 |
| S | O(6) | 2,4-Cl | 5-OMe | Et/H | CH₂OBu | 3 |
| S | S(5) | 4-Me | 6-Me | Me/Me | allyl | 2 |
| O | NMe(6) | 4-CF₃ | H | H/H | iPr | 3 |
| S | O(4) | 4-CF₃ | 5-Me | Me/Me | hexyl | 2 |
| O | O(6) | 4-CF₃ | 5-allyl | Me/Me | iPr | 2 |
| O | O(6) | 4-CF₃ | 5-allyl | H/H | iPr | 2 |

### (5) Compounds represented by the following formula:

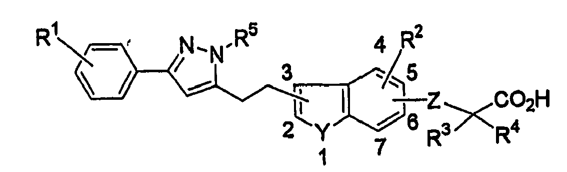

[in which Y, Z, R¹, R², R³, R⁴, and R⁵ are described in Table 6; in Table 6, Position means a substitution position of (CH₂)₂].

**Table 6**

| Y | Z | R¹ | R² | R³/R⁴ R⁵ | Position | |
|---|---|---|---|---|---|---|
| O | O(6) | 4-CF₃ | 5-OMe | H/H | OEt | 3 |
| O | O(6) | 4-Cl | 5-OMe | Me/Me | secBu | 3 |
| O | O(5) | 2-Cl, 4-CF₃ | 7-Me | Me/Me | iPr | 3 |
| O | O(6) | 4-CF₃ | 4-Me | Me/Me | heptyl | 2 |
| S | O(6) | 4-OMe | 5-Me | Me/Me | nPr | 3 |
| S | O(6) | 4-OMe | 5-Me | Me/Me | nPr | 2 |
| S | O(6) | 4-OMe | 5-Me | H/H | nPr | 3 |
| S | S(6) | 4-CF₃ | 5-Et | H/H | hexyl | 3 |

### (6) Compounds represented by the following formula:

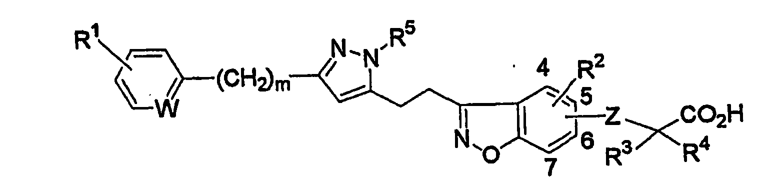

[in which W, Z, R¹, R², R³, R⁴, R⁵ and m are described in Table 7].

**Table 7**

| W | Z | R¹ | R² | R³/R⁴ R⁵ | m | |
|---|---|---|---|---|---|---|
| CH | O(6) | 4-CF₃ | 5-Me | Me/Me | iPr | 1 |
| CH | O(6) | 4-CF₃ | 5-Me | H/H | iPr | 1 |
| CH | O(6) | 4-CF₃ | 5-Me | Me/Me | iPr | 2 |
| CH | O(6) | 4-Me | 5-Me | Me/Me | iPr | 1 |
| N | O(6) | 5-CF₃ | 5-Me | Me/Me | iPr | 0 |
| N | O(5) | 5-Me | 6-Et | H/H | hexyl | 0 |
| N | O(5) | 5-Me | 7-Me | Me/Me | Me | 0 |
| N | S(6) | 3,5-Cl | 5-H | Me/Me | nBu | 0 |

### (7) Compounds represented by the following formula:

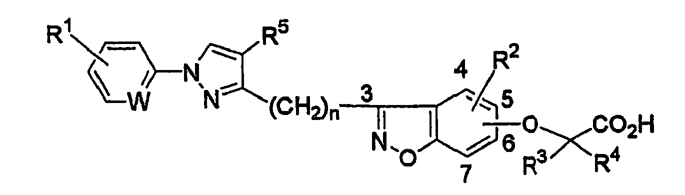

[in which W, R¹, R², R³, R⁴, R⁵ and n are described in Tables 8 and 9; in Tables 8 and 9, Position means a substitution position of O].

**Table 8**

| W | R¹ | R² | R³/R⁴ R⁵ | Position | n | |
|---|---|---|---|---|---|---|
| CH | 4-CF₃ | 5-Me | Me/Me | iPr | 6 | 2 |
| CH | 4-CF₃ | 5-Me | H/H | iPr | 6 | 2 |
| N | 5-CF₃ | 5-Me | H/H | iPr | 6 | 2 |
| CH | 4-Me | 5-Me | Me/Me | iPr | 6 | 2 |
| CH | 4-CF₃ | 5-Me | Me/Me | hexyl | 6 | 2 |
| N | 5-CF₃ | 5-Me | Me/Me | hexyl | 6 | 2 |
| CH | 4-CF₃ | 5-Me | H/H | hexyl | 6 | 2 |
| CH | 4-CF₃ | 5-Me | Me/Me | iPr | 6 | 3 |
| CH | 4-CF₃ | 5-Me | Me/Me | iPr | 6 | 4 |
| CH | 4-CF₃ | 5-Et | Me/Me | hexyl | 6 | 2 |

**Table 9**

| W | R¹ | R² | R³/R⁴ R⁵ | Position | n | |
|---|---|---|---|---|---|---|
| CH | 3,4-Cl | 7-OMe | H/H | allyl | 5 | 3 |
| CH | 4-OCF₃ | 5-Et | H/H | allyl | 6 | 2 |
| N | 5-OCF₃ | 5-Et | Me/Me | allyl | 6 | 3 |
| CH | 3-Cl, 4-CF₃ | 6-Me | Me/Me | iPr | 5 | 2 |
| CH | 3-Cl, 4-CF₃ | 6-Me | H/H | iPr | 5 | 2 |
| CH | 4-CF₃ | 6-Me | Me/Me | CH₂OBu | 5 | 2 |
| CH | 3-F,4-Cl | 6-allyl | Et/H | hexyl | 5 | 3 |
| CH | 3-F,4-Cl | 6-allyl | Et/H | hexyl | 5 | 3 |
| CH | 3-F,4-Cl | 6-allyl | Et/H | hexyl | 5 | 3 |

### (8) Compounds represented by the following formula:

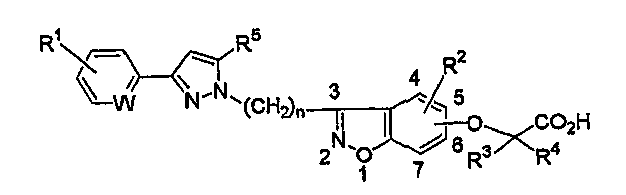

[in which W, R¹, R², R³, R⁴, R⁵ and n are described in Table 10; in Table 10, Position means a substitution position of O].

**Table 10**

| W | R¹ | R² | R³/R⁴ R⁵ | Position | n | |
|---|---|---|---|---|---|---|
| CH | 4-CF₃ | 5-Me | Me/Me | iPr | 6 | 3 |
| CH | 4-CF₃ | 5-Me | H/H | iPr | 6 | 3 |
| N | 4-CF₃ | 6-Et | Me/Me | hexyl | 5 | 3 |
| N | 4-Me | H | Me/Me | H | 4 | 3 |
| CH | 4-Me,2-F | 7-Me | H/H | Me | 6 | 4 |

### (9) Compounds represented by the following formula:

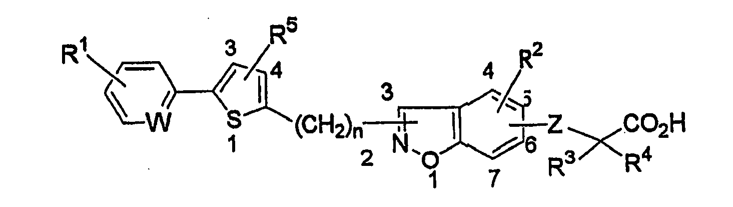

[in which W, Z, R¹, R², R³, R⁴, R⁵ and n are described in Table 11].

**Table 11**

| W | Z | R¹ | R² | R³/R⁴ R⁵ | n | |
|---|---|---|---|---|---|---|
| CH | O(6) | 4-CF₃ | 5-Me | Me/Me | 4-iPr | 2 |
| CH | O(6) | 4-CF₃ | 5-Me | H/H | 4-iPr | 2 |
| CH | O(6) | 4-CF₃ | 5-Me | Me/Me | 4-hexyl | 3 |
| CH | O(6) | 4-CF₃ | 5-Me | H/H | 4-hexyl | 2 |
| N | 0(6) | 5-CF₃ | 5-Me | Me/Me | 4-iPr | 2 |
| CH | O(5) | 4-Me | 6-Me | Me/Me | 3-hexyl | 3 |
| N | O(6) | 3,4-Cl | 7-Me | H/H | 4-Bu | 2 |
| CH | O(5) | 4-F | 6-Et | H/H | 3-allyl | 2 |
| CH | NMe(6) | 4-CF₃ | H | H/H | 4-hexyl | 2 |

### (10) Compounds represented by the following formula:

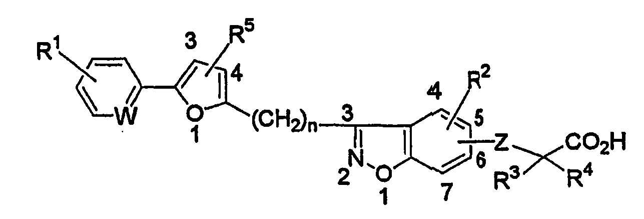

[in which W, Z, R¹, R², R³, R⁴, R⁵ and n are described in Table 12].

**Table 12**

| W | Z | R¹ | R² | R³/R⁴ R⁵ | n | |
|---|---|---|---|---|---|---|
| CH | O(6) | 4-CF₃ | 5-Me | Me/Me | 4-iPr | 2 |
| CH | O(6) | 4-CF₃ | 5-Me | H/H | 4-iPr | 2 |
| CH | O(6) | 4-CF₃ | 5-Me | Me/Me | 4-hexyl | 2 |
| CH | O(6) | 4-CF₃ | 5-Me | H/H | 4-hexyl | 2 |
| N | O(6) | 5-CF₃ | 5-Me | Me/Me | 4-iPr | 2 |
| CH | O(5) | 4-Me | 6-Me | Me/Me | 3-hexyl | 2 |
| CH | O(6) | 3,4-Cl | 7-Me | H/H | 4-Bu | 3 |
| N | S(4) | 5-CF₃ | 5-Me | Me/Me | 4-cyclopropyl | 2 |
| CH | O(5) | 4-F | 6-Et | H/H | 3-allyl | 3 |
| CH | NMe(6) | 4-CF₃ | H | H/H | 4-hexyl | 2 |

### (11) Compounds represented by the following formula:

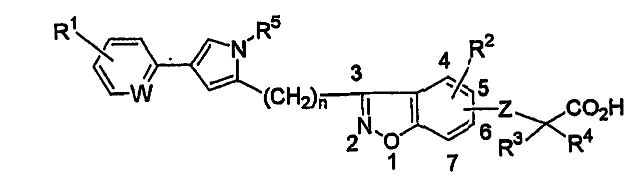

[in which W, Z, R¹, R², R³, R⁴, R⁵ and n are described in Table 13].

**Table 13**

| W | Z | R¹ | R² | R³/R⁴R⁵ | n | |
|---|---|---|---|---|---|---|
| CH | O(6) | 4-CF₃ | 5-Me | Me/Me | iPr | 2 |
| CH | O(6) | 4-CF₃ | 5-Me | H/H | iPr | 2 |
| CH | O(6) | 4-CF₃ | 5-Me | Me/Me | hexyl | 2 |
| CH | O(6) | 4-CF₃ | 5-Me | H/H | hexyl | 2 |
| N | O(6) | 5-CF3 | 5-Me | Me/Me | iPr | 2 |
| CH | O(5) | 4-Me | 6-Me | Me/Me | hexyl | 2 |
| CH | O(6) | 3,4-Cl | 7-Me | H/H | Bu | 3 |
| N | S(4) | 5-CF₃ | 5-Me | Me/Me | cyclopropyl | 2 |
| CH | 0(5) | 4-F | 6-Et | H/H | allyl | 3 |
| CH | NMe(6) | 4-CF₃ | H | H/H | hexyl | 2 |

### (12) Compounds represented by the following formula:

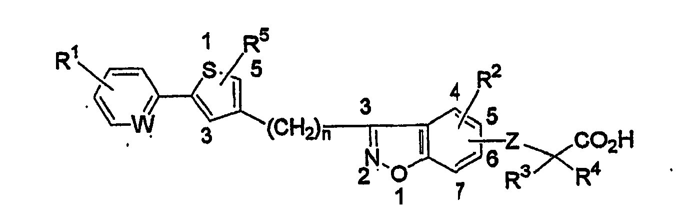

[in which W, Z, R¹, R², R³, R⁴, R⁵ and n are described in Table 14].

**Table 14**

| W | Z | R¹ | R² | R³/R⁴R⁵ | n | |
|---|---|---|---|---|---|---|
| CH | O(6) | 4-CF₃ | 5-Me | Me/Me | 5-iPr | 2 |
| CH | O(6) | 4-CF₃ | 5-Me | H/H | 5-iPr | 2 |
| CH | O(6) | 4-CF₃ | 5-Me | Me/Me | 5-hexyl | 2 |
| CH | O(6) | 4-CF₃ | 5-Me | H/H | 5-hexyl | 2 |
| N | O(6) | 5-CF₃ | 5-Me | Me/Me | 5-iPr | 2 |
| CH | O(5) | 4-Me | 6-Me | Me/Me | 3-hexyl | 2 |
| CH | O(6) | 3,4-Cl | 7-Me | H/H | 5-Bu | 3 |
| N | S(4) | 5-CF₃ | 5-Me | Me/Me | 5-cyclopropyl | 2 |
| CH | O(5) | 4-F | 6-Et | H/H | 5-allyl | 3 |
| CH | NMe(6) | 4-CF₃ | H | H/H | 5-hexyl | 2 |

### (13) Compounds represented by the following formula:

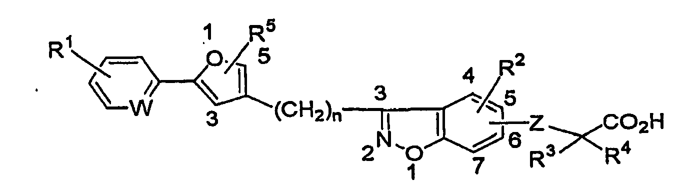

[in which W, Z, R¹, R², R³, R⁴, R⁵ and n are described in Table 15].

**Table 15**

| W | Z | R¹ | R² | R³/R⁴ R⁵ | n | |
|---|---|---|---|---|---|---|
| CH | O(6) | 4-CF₃ | 5-Me | Me/Me | 5-iPr | 2 |
| CH | O(6) | 4-CF₃ | 5-Me | H/H | 5-iPr | 2 |
| CH | O(6) | 4-CF₃ | 5-Me | Me/Me | 5-hexyl | 2 |
| CH | O(6) | 4-CF₃ | 5-Me | H/H | 5-hexyl | 2 |
| N | O(6) | 5-CF₃ | 5-Me | Me/Me | 5-iPr | 2 |
| CH | O(5) | 4-Me | 6-Me | Me/Me | 3-hexyl | 2 |
| CH | O(6) | 3,4-Cl | 7-Me | H/H | 5-Bu | 3 |
| N | S(4) | 5-CF₃ | 5-Me | Me/Me | 5-cyclopropyl | 2 |
| CH | O(5) | 4-F | 6-Et | H/H | 5-allyl | 3 |
| CH | NMe(6) | 4-CF₃ | H | H/H | 5-hexyl | 2 |

### (14) Compounds represented by the following formula:

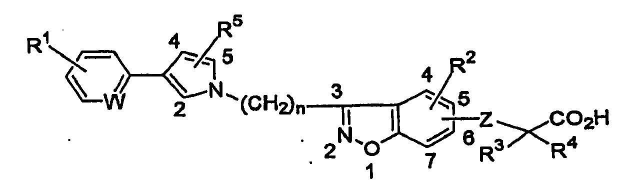

[in which W, Z, R¹, R², R³, R⁴, R⁵ and n are described in Table 16].

**Table 16**

| W | Z | R¹ | R² | R³/ R⁴ R⁵ | n | |
|---|---|---|---|---|---|---|
| CH | O(6) | 4-CF₃ | 5-Me | Me/Me | 5-iPr | 2 |
| CH | O(6) | 4-CF₃ | 5-Me | H/H | 5-iPr | 2 |
| CH | O(6) | 4-CF₃ | 5-Me | Me/Me | 5-hexyl | 2 |
| CH | O(6) | 4-CF₃ | 5-Me | H/H | 5-hexyl | 2 |
| N | O(6) | 5-CF₃ | 5-Me | Me/Me | 5-iPr | 2 |
| CH | O(5) | 4-Me | 6-Me | Me/Me | 4-hexyl | 2 |
| CH | O(6) | 3, 4-Cl | 7-Me | H/H | 5-Bu | 3 |
| N | S(4) | 5-CF₃ | 5-Me | Me/Me | 5-cyclopropyl | 2 |
| CH | O(5) | 4-F | 6-Et | H/H | 5-allyl | 3 |
| CH | NMe(6) | 4-CF₃ | H | H/H | 5-hexyl | 2 |
| CH | CH₂(6) | 4-CF₃ | 5-Et | Me/Me | 5-CH₂OBu | 2 |
| CH | O(4) | 3,4-Cl | 5-Et | H/H | 4-iPr | 3 |

The pharmacological effects of the invention are described below.

For determining PPARδ activating effect of the compounds according to the invention, PPARδ activating effects of test compounds (compounds of Examples) were measured by the following method:

A receptor expression plasmid (pSG5-GAL4-hPPAR α or γ or δ (LBD)), a luciferase expression plasmid (pUC8-MH100x4-TK-Luc) and β-galactosidase expression plasmid (pCMX-β-GAL) (Kliewer, S.A., et. al.,(1992) Nature, 358:771-774) are transfected into CV-1 cells (ATCC). After gene transfer utilizing a lipofection reagent DMRIE-C or Lipofectamin 2000 (Invitrogen), it is incubated for 42 hours in the presence of the test compound. Then, the luciferase activity and β-GAL activity are measured on the soluble cells. The luciferase activity is calibrated by the β-GAL activity. A relative ligand activity is calculated for each of the PPAR α, γ and δ under the following conditions: a relative activity of PPAR α is calculated in consideration of a luciferase activity (assigned to 100%) of cells treated with the compound GW-590735 (PPARα-selective agonist); a relative activity of PPAR γ is calculated in consideration of a luciferase activity (assigned to 100%) cells treated with Rosiglitazone; and a relative activity of PPAR δ is calculated in consideration of a luciferase activity (assigned to 100%) of cells treated with GW-501516. See the below-described Example 4.

As is apparent from Table 17, the compounds of the invention show excellent PPARδ activating effect. Since the compound of the invention having the formula (I) or (II) shows excellent PPARδ activating effect, it is expected to serve as remedy for prevention and treatment of the following diseases: hyperglycemia, obesity, syndrome X, hyperchloresterolemia, hyperlipopreoteinemia, low HDL-emia, other dysbolismic diseases, hiperlipemia, arterial sclerosis, diseases of cardiovascular systems, hyperphagia, ischemic diseases, malignant tumors such as lung cancer, mammary cancer, colonic cancer, cancer of great intestine and ovary cancer, Alzheimer's disease, and inflammatory disease.

The compound of the invention can be administered to human beings by ordinary administration methods such as oral administration or parenteral administration.

The compound can be granulated in ordinary manners for the preparation of pharmaceuticals. For instance, the compound can be processed to give pellets, granule, powder, capsule, suspension, injection, suppository, and the like.

For the preparation of these pharmaceuticals, ordinary additives such as vehicles, disintegrators, binders, lubricants, dyes, and diluents. As the vehicles, lactose, D-mannitol, crystalline cellulose and glucose can be mentioned. Further, there can be mentioned starch and carboxymethylcellulose calcium (CMC-Ca) as the disintegrators, magnesium stearate and talc as the lubricants, and hydroxypropylcellulose (HPC), gelatin and polyvinylpirrolidone (PVP) as the binders.

The compound of the invention can be administered to an adult generally in an amount of 0.1 mg to 100 mg a day by parenteral administration and 1 mg to 2,000 mg a day by oral administration. The dosage can be adjusted in consideration of age and conditions of the patient.

The invention is further described by the following non-limiting examples.

### [Example 1] 2-[3-[2-[1-Isopropyl-3-(4-trifluoromethylphenyl)-1H-pyrazol-5-yl]ethyl]-5-methylbenzisoxazol-6-yloxy]-2-methylpropionic acid

### (1) N-[3-[2-[1-Isopropyl-3-(4-trifluoromethylphenyl)-1H-pyrazol-5-yl]ethyl]-5-methylbenzisoxazol-6-yl]acetamide

To a suspension of N-(3,5-dimethylbenzisoxazol-6-yl)acetamide (30 mg, 1.466 mmol) in anhydrous tetrahydrofuran (13 mL) was dropwise added 2M LDA (1.76 mL, 3.518 mmol) at -78°C for 30 minutes in a nitrogen atmosphere. The mixture was subsequently stirred for 30 minutes at the same temperature. To the mixture was dropwise added a solution of 5-chloromethyl-1-isopropyl-3-(4-trifluoromethylphenyl)-1H-pyrazole (532 mg, 1.759 mmol) in anhydrous tetrahydrofuran (4 mL) for 30 minutes. The mixture was then stirred for one hour at the same temperature, and allowed to reach the room temperature. To the mixture were added saturated aqueous ammonium chloride and ethyl acetate. The organic portion was collected, washed with saturated brine, dried over anhydrous sodium sulfate, and placed under pressure to distill the solvent off. The residue was subjected to silica gel column chromatography and eluted with chloroform/methanol (20/1, v/v), to give the titled compound as a yellow crystalline product (268 mg, yield 32.4%).
¹H NMR (CDCl₃, 400 MHz) δ: 1.47 (6H, d, J=7Hz), 2.26 (3H, s), 2.31 (3H, s), 3.2-3.3 (2H, m), 3.3-3.4 (2H, m), 4.3-4.5 (1H, m), 6.44 (1H, s), 6.84 (1H, s), 7.25 (1H, s), 7.63 (2H, d, J=8Hz), 7.88 (2H, d, J=8Hz), 8.40 (1H, br s)

### (2) 6-Amino-3-[2-[1-isopropyl-3-(4-trifluoromethylphenyl)-1H-pyrazol-5-yl]ethyl]-5-methylbenzisoxazole

The above-mentioned N-[3-[2-[1-isopropyl-3-(4-trifluoromethylphenyl)-1H-pyrazol-5-yl]ethyl]-5-methylbenzisoxazol-6-yl]acetamide (268 mg, 0.570 mmol) was dissolved in acetic acid (15 mL) and 1.2N hydrochloric acid (7 mL), and the resulting solution was heated under reflux for 15 hours. The solution was allowed to reach the room temperature. To the solution was then added aqueous 4N sodium hydroxide (20 mL). The mixture was subjected to extraction with ethyl acetate. The organic portion was collected, washed with saturated aqueous sodium hydrogen carbonate and saturated brine, dried over anhydrous sodium sulfate, and placed under reduced pressure to distill the solvent off. The residue was subjected to silica gel column chromatography and eluted with hexane/ethyl acetate (2/1, v/v), to give the titled compound as a pale yellow amorphous product (136 mg, yield 56%).
¹H NMR (CDCl₃, 400 MHz) δ: 1.48 (6H, d, J=7Hz), 2.20 (3H, s), 3.1-3.2 (2H, m), 3.2-3.3 (2H, m), 4.02 (2H, br s), 4.3-4.5 (1H, m), 6.45 (1H, s), 6.75 (1H, s), 7.13 (1H, s), 7.61 (2H, d, J=8Hz), 7.89 (2H, d, J=8Hz)

### (3) 3-[2-[1-Isopropyl-3-(4-trifluoromethylphenyl)-1H-pyrazol-5-yl]ethyl]-6-hydroxy-5-methylbenzisoxazole

The above-mentioned 6-amino-3-[2-[1-isopropyl-3-(4-trifluoromethylphenyl)-1H-pyrazol-5-yl]ethyl]-5-methylbenzisoxazole (130 mg, 0.303 mmol) was suspended in aqueous 25% sulfuric acid (2.3 mL). To the resulting suspension was dropwise added an aqueous solution (1 mL) containing sodium sulfite (31 mg, 0.455 mmol) under icecooling. The mixture was stirred for one hour. To the mixture was then dropwise added aqueous 75% sulfuric acid (heated to 120°C) for 5 minutes. The resulting mixture was heated for 2.5 hours at the same temperature and then allowed to reach the room temperature. After addition of water, the mixture was subjected to extraction with ethyl acetate. The organic portion was collected, washed with saturated brine and water, dried over anhydrous sodium sulfate, and placed under reduced pressure to distill the solvent off. The residue was subjected to silica gel column chromatography and eluted with chloroform, to give the titled compound as a yellow crystalline product (83 mg, yield 64%).
¹H NMR (CDCl₃, 400 MHz) δ: 1.48 (6H, d, J=6Hz), 2.30 (3H, s), 3.1-3.2 (2H, m), 3.2-3.3 (2H, m), 4.3-4.5 (1H, m), 5.54 (1H, br s), 6.46 (1H, s), 6.94 (1H, s), 7.22 (1H, s), 7.61 (2H, d, J=8Hz), 7.88 (2H, d, J=8Hz)

### (4) Ethyl 2-[3-[2-[1-isopropyl-3-(4-trifluoromethylphenyl)-1H-pyrazol-5-yl]ethyl]-5-methylbenzisoxazol-6-yloxy]-2-methylpropionate

The above-mentioned 3-[2-[1-isopropyl-3-(4-trifluoromethylphenyl)-1H-pyrazol-5-yl]ethyl]-6-hydroxy-5-methylbenzisoxazole (40 mg, 0.0931 mmol) and potassium carbonate (64 mg, 0.463 mmol) were suspended in 2-butanone (2 mL). To the resulting suspension was added ethyl 2-bromo-2-methylpropionate (0.07 mL, 0.466 mmol). The mixture was heated under reflux for 16 hours and allowed to reach the room temperature. After addition of saturated aqueous ammonium chloride, the mixture was subjected to extraction with ethyl acetate. The residue was subjected to silica gel column chromatography and eluted with hexane/ethyl acetate (8/1 to 5/1, v/v), to give the titled compound as a colorless oil (34 mg, yield 67%) .
¹H NMR (CDCl₃, 400 MHz) δ: 1.24 (3H, t, J=7Hz), 1.48 (6H, d, J=6Hz), 1.68 (6H, s), 2.27 (3H, s), 3.1-3.2 (2H, m), 3.2-3.3 (2H, m), 4.25 (2H, q, J=7Hz), 4.3-4.5 (1H, m), 6.45 (1H, s), 6.78 (1H, s), 7.22 (1H, s), 7.61 (2H, d, J=8Hz), 7.89 (2H, d, J=8Hz)

### (5) 2-[3-[2-[1-Isopropyl-3-(4-trifluoromethylphenyl)-1H-pyrazol-5-yl]ethyl]-5-methylbenzisoxazol-6-yloxy]-2-methylpropionic acid

The above-mentioned ethyl 2-[3-[2-[1-isopropyl-3-(4-trifluoromethylphenyl)-1H-pyrazol-5-yl]ethyl]-5-methylbenzisoxazol-6-yloxy]-2-methylpropionate (30 mg, 0.0552 mmol) was suspended in a mixture of ethanol (2 mL) and water (1 mL). After addition of lithium hydroxide monohydrate (7 mg, 0.166 mmol), the suspension was heated under reflux for one hour, and then allowed to reach the room temperature. The suspension was made acidic by addition of ice-water and 1N hydrochloric acid, and subjected to extraction with ethyl acetate. The organic portion was collected, washed with saturated brine, dried over anhydrous sodium sulfate, and placed under reduced pressure to distill the solvent off. The residue was dried under reduced pressure, to give the titled compound as a colorless amorphous product (34 mg, yield >99%).
FAB-MS (m/e): 516 (M+1)
¹H NMR (CDCl₃, 400 MHz) δ: 1.46 (6H, d, J=6Hz), 1.69 (6H, s), 2.27 (3H, s), 3.1-3.2 (2H, m), 3.2-3.3 (2H, m), 4.3-4.5 (1H, m), 6.44 (1H, s), 6.91 (1H, s), 7.24 (1H, s), 7.60 (2H, d, J=8Hz), 7.87 (2H, d, J=8Hz)

### [Example 2] 3-[2-[1-Isopropyl-3-(4-trifluoromethylphenyl)-1H-pyrazol-5-yl]ethyl]-5-methylbenzisoxazol-6-yloxyacetic acid

### (1) Ethyl 3-[2-[1-isopropyl-3-(4-trifluoromethylphenyl)-1H-pyrazol-5-yl]ethyl]-5-methylbenzisoxazol-6-yloxyacetate

The 3-[2-[1-isopropyl-3-(4-trifluoromethylphenyl)-1H-pyrazol-5-yl]ethyl]-6-hydroxy-5-methylbenzisoxazole (40 mg, 0.0931 mmol) obtained in Example 1-(3) and potassium carbonate (40 mg, 0.279 mmol) were suspended in acetone (2 mL). To the resulting suspension was added ethyl bromoacetate (0.03 mL, 0.279 mmol) under ice-cooling. The mixture was stirred for 3.5 hours under the same conditions. After addition of saturated aqueous ammonium chloride, the mixture was subjected to extraction with ethyl acetate. The organic portion was collected, washed with saturated brine, and dried over anhydrous sodium sulfate. The solvent was then distilled off. The residue was subjected to silica gel column chromatography and eluted with hexane/ethyl acetate (7/1 to 4.1, v/v), to give the titled compound as a white crystalline product (30 mg, yield 63%).
¹H NMR (CDCl₃, 400 MHz) δ: 1.31 (3H, t, J=7Hz), 1.49 (6H, d, J=7Hz), 2.33 (3H, s), 3.1-3.3 (2H, m), 3.3-3.4 (2H, m), 4.29 (2H, q, J=7Hz), 4.4-4.5 (1H, m), 4.72 (2H, s), 6.45 (1H, s), 6.84 (1H, s), 7.25 (1H, s), 7.61 (2H, d, J=8Hz), 7.89 (2H, d, J=8Hz)

### (2) 3-[2-[1-Isopropyl-3-(4-trifluoromethylphenyl)-1H-pyrazol-5-yl]ethyl]-5-methylbenzisoxazol-6-yloxyacetic acid

Procedures similar to those of Example 1-(5) were carried out to give the title compound.
White crystalline product
Yield 88%
FAB-MS (m/e): 488 (M+1)
¹H NMR (CDCl₃, 400 MHz) δ: 1.48 (6H, d, J=6Hz), 2.33 (3H, s), 3.1-3.2 (2H, m), 3.2-3.3 (2H, m), 4.4-4.5 (1H, m), 4.72 (2H, s), 6.45 (1H, s), 6.88 (1H, s), 7.26 (1H, s), 7.62 (2H, d, J=8Hz), 7.88 (2H, d, J=8Hz)

### [Example 3] 2-[3-[2-[3-Isopropyl-1-(4-trifluoromethylphenyl)-1H-pyrazol-4-yl]ethyl]-5-methylbenzisoxazol-6-yloxy]-2-methylpropionic acid

### (1) N-[3-[2-[3-Isopropyl-1-(4-trifluoromethylphenyl)-1H-pyrazol-4-yl]ethyl]-5-methylbenzisoxazol-6-yl]acetamide

The procedures of Example 1-(1) were repeated using 4-chloromethyl-3-isopropyl-1-(4-trifluoromethylphenyl)-1H-pyrazole, to give the titled compound.
Pale yellow amorphous product
Yield 16%
¹H NMR (CDCl₃, 400 MHz) δ: 1.34 (6H, d, J=7Hz), 2.26 (3H, s), 2.31 (3H, s), 3.0-3.1 (3H, m), 3.2-3.3 (2H, m), 7.08 (1H, s), 7.27 (1H, s), 7.64 (2H, d, J=9Hz), 7.68 (1H, s), 7.71 (2H, d, J=9Hz), 8.39 (1H, br s)

### (2) 6-Amino-3-[2-[3-isopropyl-1-(4-trifluoromethylphenyl)-1H-pyrazol-4-yl]ethyl]-5-methylbenzisoxazole

The procedures of Example 1-(2) were repeated using the above-mentioned N-[3-[2-[3-isopropyl-1-(4-trifluoromethylphenyl)-1H-pyrazol-4-yl]ethyl]-5-methylbenzisoxazol-6-yl]acetamide, to give the titled compound.
Pale brown amorphous product
Yield 74%
¹H NMR (CDCl₃, 400 MHz) δ: 1.33 (6H, d, J=7Hz), 2.21 (3H, s), 3.0-3.1 (3H, m), 3.1-3.2 (2H, m), 3.99 (2H, br s), 6.76 (1H, s), 7.16 (1H, s), 7.64 (2H, d, J=8Hz), 7.71 (1H, s), 7.72 (2H, d, J=8Hz)

### (3) 3-[2-[3-Isopropyl-1-(4-trifluoromethylphenyl)-1H-pyrazol-4-yl]ethyl]-6-hydroxy-5-methylbenzisoxazole

The procedures of Example 1-(3) were repeated using the above-mentioned 6-amino-3-[2-[3-isopropyl-1-(4-trifluoromethylphenyl)-1H-pyrazol-4-yl]ethyl]-5-methylbenzisoxazole, to give a crude product of the titled compound.
¹H NMR (CDCl₃, 400 MHz) δ: 1.33 (6H, d, J=7Hz), 2.30 (3H, s), 2.9-3.1 (3H, m), 3.2-3.3 (2H, m), 5.58 (1H, br s), 6.93 (1H, s), 7.24 (1H, s), 7.63 (2H, d, J=8Hz), 7.72 (1H, s), 7.73 (2H, d, J=8Hz)

### (4) Ethyl 2-[3-[2-[3-isopropyl-1-(4-trifluoromethylphenyl)-1H-pyrazol-4-yl]ethyl]-5-methylbenzisoxazol-6-yloxy]-2-methylpropionate

The procedures of Example 1-(4) were repeated using the above-mentioned crude product of 3-[2-[3-isopropyl-1-(4-trifluoromethylphenyl)-1H-pyrazol-4-yl]ethyl]-6-hydroxy-5-methylbenzisoxazole, to give the titled compound.
Colorless oil
Yield 35% (two steps)
¹H NMR (CDCl₃, 400 MHz) δ: 1.24 (3H, t, J=7Hz), 1.33 (6H, d, J=7Hz), 1.67 (6H, s), 2.26 (3H, s), 2.9-3.1 (3H, m), 3.1-3.3 (2H, m), 4.25 (2H, q, J=7Hz), 6.77 (1H, s), 7.24 (1H, s), 7.64 (2H, d, J=8Hz), 7.71 (1H, s), 7.72 (2H, d, J=8Hz)

### (5) 2-[3-[2-[3-Isopropyl-1-(4-trifluoromethylphenyl)-1H-pyrazol-4-yl]ethyl]-5-methylbenzisoxazol-6-yloxy]-2-methylpropionic acid

Procedures similar to those of Example 1-(5) were carried out, to give the titled compound.
Pale yellow amorphous product
Quantitative yield
FAB-MS (m/e): 516 (M+1)
¹H NMR (CDCl₃, 400 MHz) δ: 1.32 (6H, d, J=7Hz), 1.72 (6H, s), 2.27 (3H, s), 2.9-3.1 (3H, m), 3.1-3.3 (2H, m), 6.92 (1H, s), 7.26 (1H, s), 7.64 (2H, d, J=9Hz), 7.71 (2H, d, J=9Hz), 7.70 (1H, s)

### [Example 4] Pharmacological Experimental

### I. Procedures of Experimental

The PPARδ activating effects of test compounds (compounds of Examples) were measured by the following method:

A receptor expression plasmid (pSG5-GAL4-hPPAR α or γ or δ (LBD)), a luciferase expression plasmid (pUC8-MH100x4-TK-Luc) and β-galactosidase expression plasmid (pCMX-β-GAL) (Kliewer, S.A., et. al.,(1992) Nature, 358:771-774) are transfected into CV-1 cells (ATCC). After gene transfer utilizing a lipofection reagent DMRIE-C or Lipofectamin 2000 (Invitrogen), it is incubated for approx. 40 hours in the presence of the test compound. Then, the luciferase activity and β-GAL activity are measured on the soluble cells. The luciferase activity is calibrated by the β-GAL activity. A relative ligand activity is calculated for each of the PPAR α, γ and δ under the following conditions: a relative activity of PPAR α is calculated in consideration of a luciferase activity (assigned to 100%) of cells treated with GW-590735 (PPARα-selective agonist); a relative activity of PPAR γ is calculated in consideration of a luciferase activity (assigned to 100%) cells treated with Rosiglitazone; and a relative activity of PPAR δ is calculated in consideration of a luciferase activity (assigned to 100%) of cells treated with GW-501516.

### II. Experimental Results of

Experimental Results are set forth in Table 17.

**Table 17**

| Test compound | PPAR α | PPAR γ | PPAR δ |
|---|---|---|---|
| Example 1 | 62.7% | 5.4% | 84.7% |
| Example 2 | 103.0 | 13.4 | 94.4 |
| Example 3 | 0.2 | 0.3 | 87.8 |

PPAR activity: relative value (%) of the test compound (10⁻⁶M) to 100% of the control compound
α: GW-590735 - 10⁻⁶ M
γ: Rosiglitazone - 10⁻⁵ M
δ: GW-501516 - 10⁻⁷ M

As is clear from Table 17, the compounds of Examples show excellent PPAR δ-activating effect.

## Claims

1. A compound having the following formula (I) or a salt thereof: 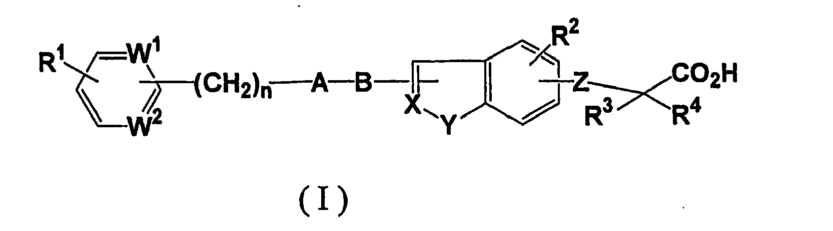 in which
each of W¹ and W² independently represents a nitrogen atom or CH,
X represents a nitrogen atom or CH,
Y represents an oxygen atom or a sulfur atom,
Z represents a bond, an oxygen atom, a sulfur atom
or NR⁵, in which R⁵ represents a hydrogen atom or an alkyl group having 1 to 8 carbon atoms,
each of R¹ and R² independently represents a hydrogen atom, a halogen atom, a hydroxyl group, a nitro group, an amino group, an alkyl group having 1 to 8 carbon atoms, a 3- to 7-membered cycloalkyl group, an alkenyl group having 2 to 8 carbon atoms, an alkynyl group having 2 to 8 carbon atoms, an alkoxy group having 1 to 8 carbon atoms, an alkyl group having 1 to 8 carbon atoms and a 3- to 7-membered cycloalkyl substituent, an alkyl group having 1 to 8 carbon atoms and a halogen substituent, an alkoxy group having 1 to 8 carbon atoms and a halogen substituent, an aryl group having 6 to 10 carbon atoms, 5- or 6-membered heterocyclic group, an aralkyl group having an aryl moiety of 6 to 10 carbon atoms and an alkylene moiety of 1 to 8 carbon atoms, or an alkyl group having 1 to 8 carbon atoms and a 5- or 6-membered heterocyclic substituent,
each of R³ and R⁴ independently represents a hydrogen atom, an alkyl group having 1 to 8 carbon atoms, or an alkyl group having 1 to 8 carbon atoms and a halogen substituent,
A represents a 5-membered hetero ring selected from the group consisting of pyrazole, thiophene, furan, isoxazole, isothiazole and pyrrole, in which the 5-membered hetero ring may have a substituent selected from the group consisting of a halogen atom, a hydroxyl group, a nitro group, an amino group, an alkyl group having 1 to 8 carbon atoms, a 3- to 7-membered cycloalkyl group, an alkenyl group having 2 to 8 carbon atoms, an alkynyl group having 2 to 8 carbon atoms, an alkoxy group having 1 to 8 carbon atoms, an alkyl group having 1 to 8 carbon atoms and a 3- to 7-membered cycloalkyl substituent, an alkyl group having 1 to 8 carbon atoms and a halogen substituent, an alkoxy group having 1 to 8 carbon atoms and a halogen substituent, an aryl group having 6 to 10 carbon atoms, a 5- or 6-membered heterocyclic group, an aralkyl group having an aryl moiety of 6 to 10 carbon atoms and an alkylene moiety of 1 to 8 carbon atoms, and an alkyl group having 1 to 8 carbon atoms and a 5- or 6-membered heterocyclic substituent,
B represents a bond or an alkylene chain having 1 to 8 carbon atoms which may have a substituent selected from the group consisting of an alkyl group having 1 to 8 carbon atoms, a 3- to 7-membered cycloalkyl group, an alkoxy group having 1 to 8 carbon atoms and a halogen substituent and further may have a double or triple bond, and
n is an integer of 0 to 3.

2. The compound or a salt thereof according to claim 1, in which each of W¹ and W² represents CH.

3. The compound or a salt thereof according to claim 1, in which W¹ represents CH and W² represents a nitrogen atom.

4. The compound or a salt thereof according to any one of claims 1 to 3, in which X represents a nitrogen atom.

5. The compound or a salt thereof according to any one of claims 1 to 3, in which X represents a nitrogen atom and Y represents an oxygen atom.

6. The compound or a salt thereof according to any one of claims 1 to 3, in which X represents CH and Y represents an oxygen atom.

7. The compound or a salt thereof according to any one of claims 1 to 6, in which Z represents an oxygen atom or a sulfur atom.

8. The compound or a salt thereof according to any one of claims 1 to 7, in which each of R¹ and R² independently represents a hydrogen atom, a halogen atom, a hydroxyl group, a nitro group, an amino group, an alkyl group having 1 to 8 carbon atoms, an alkoxy group having 1 to 8 carbon atoms, an alkyl group having 1 to 8 carbon atoms and a halogen substituent, or an alkoxy group having 1 to 8 carbon atoms and a halogen substituent.

9. The compound or a salt thereof according to any one of claims 1 to 8, in which each of R³ and R⁴ independently represents a hydrogen atom or an alkyl group having 1 to 8 carbon atoms.

10. The compound or a salt thereof according to any one of claims 1 to 9, in which A represents pyrazole, thiophene or furan which may have a substituent selected from the group consisting of a halogen atom, a hydroxyl group, a nitro group, an amino group, an alkyl group having 1 to 8 carbon atoms, a 3- to 7-membered cycloalkyl group, an alkenyl group having 2 to 8 carbon atoms, an alkynyl group having 2 to 8 carbon atoms, an alkoxy group having 1 to 8 carbon atoms, an alkyl group having 1 to 8 carbon atoms and a 3- to 7-membered cycloalkyl substituent, an alkyl group having 1 to 8 carbon atoms and a halogen substituent, an alkoxy group having 1 to 8 carbon atoms and a halogen substituent, an aryl group having 6 to 10 carbon atoms, a 5- or 6-membered heterocyclic group, an aralkyl group having an aryl moiety of 6 to 10 carbon atoms and an alkylene moiety of 1 to 8 carbon atoms, and an alkyl group having 1 to 8 carbon atoms and a 5- or 6-membered heterocyclic substituent.

11. The compound or a salt thereof according to any one of claims 1 to 9, in which A represents pyrazole, thiophene or furan which may have a substituent selected from the group consisting of a halogen atom, a hydroxyl group, a nitro group, an amino group, an alkyl group having 1 to 8 carbon atoms, an alkoxy group having 1 to 8 carbon atoms, an alkyl group having 1 to 8 carbon atoms and a halogen substituent, or an alkoxy group having 1 to 8 carbon atoms and a halogen substituent.

12. The compound or a salt thereof according to any one of claims 1 to 9, in which A represents pyrazole which may have a substituent selected from the group consisting of a halogen atom, a hydroxyl group, a nitro group, an amino group, an alkyl group having 1 to 8 carbon atoms, an alkoxy group having 1 to 8 carbon atoms, an alkyl group having 1 to 8 carbon atoms and a halogen substituent, and an alkoxy group having 1 to 8 carbon atoms and a halogen substituent.

13. The compound or a salt thereof according to any one of claims 1 to 12, in which B represents an alkylene chain having 2 to 4 carbon atoms.

14. The compound or a salt thereof according to any one of claims 1 to 13, in which n is 0.

15. A compound having the following formula (II) or a salt thereof: 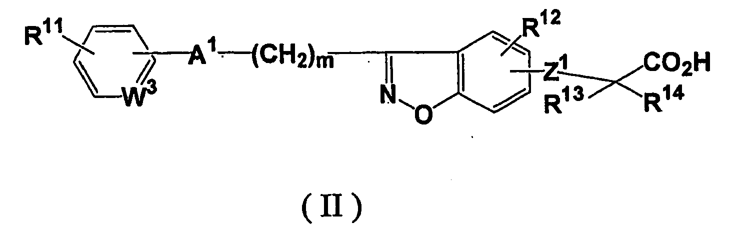 in which
W³ represents a nitrogen atom or CH,
Z¹ represents an oxygen atom or a sulfur atom,
each of R¹¹ and R¹² independently represents a hydrogen atom, a halogen atom, a hydroxyl group, a nitro group, an amino group, an alkyl group having 1 to 8 carbon atoms, an alkoxy group having 1 to 8 carbon atoms, an alkyl group having 1 to 8 carbon atoms and a halogen substituent, or an alkoxy group having 1 to 8 carbon atoms and a halogen substituent,
each of R¹³ and R¹⁴ independently represents a hydrogen atom or an alkyl group having 1 to 8 carbon atoms,
A¹ represents pyrazole or thiophene which may have a substituent selected from the group consisting of a halogen atom, a hydroxyl group, a nitro group, an amino group, an alkyl group having 1 to 8 carbon atoms, an alkoxy group having 1 to 8 carbon atoms, an alkyl group having 1 to 8 carbon atoms and a halogen substituent, or an alkoxy group having 1 to 8 carbon atoms and a halogen substituent, and
m is an integer of 2 to 4.

16. The compound or a salt thereof according to claim 15, in which W³ represents CH.

17. The compound or a salt thereof according to claim 15 or 16, in which A¹ represents pyrazole which may have a substituent selected from the group consisting of a halogen atom, a hydroxyl group, a nitro group, an amino group, an alkyl group having 1 to 8 carbon atoms, an alkoxy group having 1 to 8 carbon atoms, an alkyl group having 1 to 8 carbon atoms and a halogen substituent, or an alkoxy group having 1 to 8 carbon atoms and a halogen substituent.

18. The compound or a salt thereof according to any one of claims 15 to 17, in which m is 2 or 3.

19. An activator for peroxisome proliferator activated receptor δ containing a compound or a salt thereof according to any one of claims 1 to 18 as an effective component.
